# EUROPEAN PATENT SPECIFICATION

(11) **EP 1 745 678 B1**
(45) Date of publication and mention of the grant of the patent: **31.08.2016**
(21) Application number: 05737543.8
(22) Date of filing: 13.05.2005
(51) Int. Cl.: H05B 6/02

(54) **INDUCTION HEATING APPARATUS FOR DISSEMINATION OF VOLATILE LIQUIDS**
INDUKTIONSHEIZVORRICHTUNG ZUR ABGABE FLÜCHTIGER FLÜSSIGKEITEN
APPAREIL DE CHAUFFAGE PAR INDUCTION PERMETTANT LA DISSEMINATION DE LIQUIDES VOLATILS

(30) Priority: 14.05.2004 GB 0410777
(43) Date of publication of application: 24.01.2007
(73) Proprietor: Givaudan SA, 1214 Vernier (CH)
(72) Inventor: BROWN, Colin, Bracknell Berkshire RG42 2BD (GB); NAISH, Guy Edward, Bicester Oxfordshire OX26 3WE (GB); STEDMAN, Andrew Philip Wilson, Haslemere Surrey GU27 1BA (GB)
(74) Representative: Givaudan Patents
(86) International application number: PCT/CH2005/000271
(87) International publication number: WO 2005/112510

(56) References cited:
- US-A1- 2003 230 567

## Description

This invention relates to a method of heating substances that are to be applied to surfaces or for dissemination of volatile liquids into an atmosphere, and to apparatus for use in this method.

There are many types of material that are applied to surfaces for aesthetic or beneficial effects. If the surface is a person's body, these may include depilatory waxes, face packs, shaving creams and gels, hair conditioning oils and aromatherapy skin oils. Many of these materials are better applied hot - indeed, some are solids and have to be melted. Other types of surfaces in which substances to be applied thereto for aesthetic or beneficial reasons may advantageously be applied hot include furniture (e.g., polishes and waxes), kitchen surfaces (e.g., cleaners), floors (e.g., polishes and insecticides) and textiles (e.g., stain removers).

There are also known in the art many apparatus for disseminating volatile liquids into an atmosphere. Such liquids include fragrances, insecticides, fungicides and medicaments. In some of these apparatus, the dissemination has been assisted by heating. In one typical apparatus, the dissemination element is a porous wick that contacts the liquid in a reservoir at one end and is open to the atmosphere at the other. At the open end, there is located a heating element, that typically takes the form of a annular heating coil surrounding part of the wick, such that its heat causes accelerated evaporation, and dissemination is assisted by convection currents. In another typical apparatus, the wick is exposed to a small electric fan that blows on the wick, again causing accelerated evaporation and additionally assisting the dissemination of liquid into an atmosphere.

While such volatile liquid dissemination apparatus function generally adequately and have been commercially successful, they are not entirely free of drawbacks. Most of these relate to potential hazards. In the existing apparatus, the devices are refillable by the insertion of a refill comprising a reservoir of liquid. The act of doing so exposes the user to the electrical circuit. In addition, there is the possibility of spillage of liquid on the circuit, with the potential of damage or fire.

It has now been found that the problems can be overcome and that it is possible to supply a substance in a form that can be heated easily and conveniently and without possibility of spillage. The invention therefore provides a method of disseminating to where it is required a substance that is advantageously disseminated or applied after undergoing a heat-induced change of state or fluidification, comprising the provision of the substance in a container that comprises a secondary induction coil, the container being adapted to be used in association with an apparatus provided with a primary induction coil, the coils being in a heat-providing relationship when the container is in association with the apparatus, and providing electricity to the primary induction coil to generate state- or fluidification-inducing heat in the secondary induction coil.

The invention additionally provides an apparatus adapted to heat substances that advantageously undergo a heat-induced change of state or fluidification for useful application, the apparatus comprising a container for holding the substance, this container additionally comprising a secondary induction coil, heating being effected by electromagnetic induction by means of a primary induction coil located in another part of the apparatus.

The principle of induction heating is, of course, well known, and a number of practical embodiments have been suggested. Examples of such embodiments include the beverage warmer of United States Patent 6,314,867 the portable hotplate of United States Patent No. 4,996,405 and the sterilising unit of US publication 2003/0230567. However, there is no suggestion in the art that this method can be usefully used for the kind of substances hereinabove described, especially for a single apparatus that can be used for a multiplicity of such substances.

In relation to this invention, the expression "heat-induced change of state" includes the classical solid-liquid, liquid-gas and solid-gas transitions. Thus, in the case of air fresheners for example, a liquid material will be converted to gaseous or vapour form. By "heat-induced fluidification" is meant the rendering more pliable and fluid of a material that is relatively non-pliable and non-fluid at normal temperatures, but that becomes more pliable and fluid at higher temperatures. Examples of such substances include thick creams and pastes, waxes and viscous liquids. It also includes the conversion of a liquid to an aerosol of fine, airborne droplets.

The container may be any suitable container for the desired substance, and it may be made of any suitable material, such as metal, ceramics, glass or plastics, the last-named being especially preferred for ease of production. The material of the container must naturally be chosen bearing in mind the service temperature to which the container will be exposed - for example, some plastics may not be suitable for higher temperature uses.

The container additionally comprises a secondary induction coil. This may be any suotable coil known to the art. A typical example is a closed coil of wire, which may either be secured to the container or simply placed on the bottom of the container and kept there by gravity. Secondary coil materials with relatively high resistivity (e.g. tungsten, steel & tin) will heat up more quickly than will low resistivity materials (e.g. aluminium, brass & copper). Magnetic materials are easier to heat than non-magnetic and the thinner the material the easier it is to heat. The preferred secondary coil is a steel coil formed from very thin wire.

In the case of solid substances that require softening or melting, a top is generally required only for transit or storage; in use, the container is open, so that the substance can be removed. In the case of liquids to be evaporated, such as fragrances or insecticides, the top of the container may advantageously be covered in use by a semi-permeable membrane, so that the liquid cannot spill, even if the container is knocked over, but that vaporised liquid can be released.

The container is designed to be used in conjunction with another part of the apparatus that comprises a primary induction coil. Provided that the two coils are placed with respect to each other such that a current in the primary coil will induce sufficient current in the secondary coil to provide the necessary degree of heating to bring about a desired change in the state or fluidification of the substance contained therein, the physical arrangement of container and the other part is not critical, and there are many possible alternatives that will be evident to the skilled person. In a preferred embodiment, the container is a separate component and can be easily removed and replaced. This will be further discussed hereinunder.

In another preferred embodiment, the other part of the apparatus takes the form of a base on which the container sits. This is a particularly convenient and preferred embodiment and the description will henceforth refer exclusively to this embodiment, although the skilled person will recognise that many other constructions falling within the scope of this invention are possible.

The container may be secured to the base in any suitable manner. A particularly preferred embodiment comprises the provision in the container of a central re-entrant portion extending upwards from the bottom of the container, the base comprising a member that extends upwards into the re-entrant portion, thus holding the container in place, yet allowing easy removal. The secondary induction coil is preferably wound around the re-entrant portion on the inside of the container and the primary induction coil is placed within the upwardly-extending base member.

The arrangement and characteristics of induction coils are well known to the art and a skilled person can easily select a suitable pairing of primary and secondary coils for any desired substance and end-use. The induced current will be influenced by (a) the relative size of the primary coil with respect to the secondary coil, and (b) the relative positioning of the coils. In the former case, the size of coil may be chosen to deliver the desired induced current. In the latter case, provision may be made for the container to be raised and lowered with respect to the primary coil, and this provides a means of temperature regulation. Alternatively, a movable primary induction coil may be raised or lowered with respect to a fixed secondary coil.

As hereinabove mentioned, in an especially preferred embodiment, the container is removable and replaceable. One of the advantages of the present invention is that containers may easily be removed and replaced without there being any danger of spillage. In a further preferment, the nature and positioning of the coils within individual containers can be varied to suit the particular application. For example, a substance requiring a higher temperature for a change of state may have a secondary coil that becomes hotter. Thus, a single base may be used for a variety of different applications. The invention therefore also provides a method of providing in usable form by means of a single apparatus a multiplicity of different substances that are advantageously disseminated or applied after undergoing a heat-induced change of state or fluidification, comprising the provision of each substance in a container that comprises a secondary induction coil, the container being adapted to be used in association with an apparatus provided with a primary induction coil, the primary and secondary induction coils being in a heat-providing relationship when the container is in association with the apparatus, and providing electricity to the primary induction coil to generate sufficient heat in the secondary induction coil to induce in the liquid a desired change of state or fluidification, the natures and/or positions of the coils being such that sufficient heat for the desired change in each individual substance is generated.

As mentioned hereinabove, the relative positions of the individual combinations of primary and secondary coils to provide the appropriate state- or fluidification-inducing heating for a particular substance may be achieved by any convenient method. One particular example is the provision of position-varying means of one coil with respect to the other, typically by raising or lowering the container. Alternatively, the primary coil may be able to be raised and lowered with respect to a stationary secondary coil. A preferred embodiment comprises the provision of a fixed primary induction coil and within each container a secondary induction coil whose nature and position is such that, in combination with the primary coil, , the state- or fluidification-inducing heating appropriate to the particular substance is provided.

In many embodiments of the apparatus, it is desirable or even essential to restrict the maximum temperature attainable by the secondary coil, for example, for reasons of efficient operation or safety. For example, in the case of an evaporable liquid, as the liquid level falls, the secondary coil may become exposed to the atmosphere, permitting it to heat up to an extent greater than that possible when it was fully immersed. One example of a mechanism to avoid this problem includes a thermal fuse connected in series in the coil, which would melt when a pre-determined temperature was reached, thus breaking the coil. Another example is the inclusion of a PTC (positive temperature coefficient) device in the base, located so that it is as close as possible to the coil in the container. Such a device experiences a rapid rise in resistance above a certain temperature, thus reducing the current in the primary coil and therefore the ability to cause heat generation in the secondary coil. A further embodiment is the incorporation of a bimetallic switch that moves and cuts the circuit above a pre-determined temperature, again preventing excessive temperature rise. The skilled person will readily appreciate that there are many other possibilities of achieving the same end and that these are all within the skill of the art and the scope of this invention.

All of the embodiments described hereinabove are easily attainable by the skilled person, using only the ordinary skill of the art.

The number of different applications for which this invention can be used is considerable. They include (but are not limited to); air fresheners for home and automobile use, aromatherapy products. depilatory waxes, insecticides and other pest control products, stain removal products, cleaning materials, creams and oils for application to skin and hair, shaving products and polishes for shoes, furniture and other surfaces. All of these varied uses can be accommodated using a single base and a variety of specialised containers. Such containers may include modifications necessary for their particular functions. For example, where the heated product needs to be applied to a surface, the container may have an associated applicator, such as a brush, a pad or a sponge. When the substance is to be disseminated into an atmosphere, the contents may be retained by means of a vapour-permeable, but liquid-impermeable microporous membrane, such as a PTFE-based membrane, for example, the types sold under the brand name Gore-Tex^{®}.

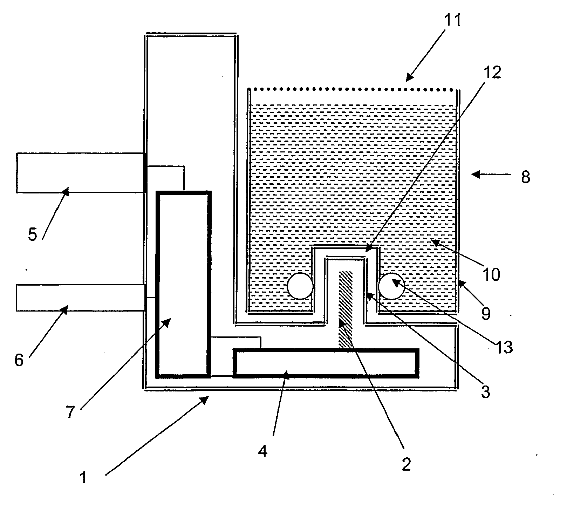

The invention is further described with reference to the accompanying drawing, which depicts a preferred embodiment and which should not be regarded as limiting.

The drawing depicts a schematic longitudinal cross-section of a preferred embodiment, which is used for the dissemination of volatile liquids into an atmosphere. A base module 1 contains a primary induction coil 2 contained within an upwardly-projecting cylindrical portion 3 of the base module.. The induction coil 2 is controlled by a driver circuit 4 connected to mains electricity supply pins 5 & 6 via a power adaptor 7. Mounted on the base unit is a refill unit 8. This comprises a reservoir 9 containing a volatile liquid 10 sealed under a porous membrane 11. The base of the reservoir has a re-entrant portion 12 that fits over the upwardly-projecting cylindrical portion 3 on the base module 1. Around the re-entrant portion 12 is placed a secondary induction coil 13 which is composed of a short-circuited wire coil that is coated with lacquer or other suitable material to protect it from the volatile liquid 10.

When electricity is passed through the primary induction coil 2, a current is induced in the secondary induction 13. This causes the coil to heat up and thus to heat the liquid 10. This evaporates and passes into the atmosphere through the porous membrane 11. The porosity of this membrane is such that the vapour can pass through, but liquid cannot. Thus, should the apparatus be knocked over, there will be no spillage with its attendant hazards, such as fire.

## Claims

1. A method of disseminating to where it is required a substance (10) that is advantageously disseminated or applied after undergoing a heat-induced change of state or fluidification, the heating being supplied by induction heating, by providing electricity to a primary induction coil (2) to generate state- or fluidification-inducing heat in a secondary induction coil (13), **characterised in that** the substance is provided in a container (8) that comprises the secondary induction coil, the container being removably or replacably seated on a base (1) which comprises the primary induction coil, the coils being in a heat-providing relationship when the container is seated on the base.

2. A method according to claim 1, in which the container is provided with a re-entrant portion (12) and the base comprises an upwardly-extending member (3) that fits into the re-entrant portion in a container-holding relationship,

3. A method according to claim 1, in which the substance is evaporable and the container comprises a semi-permeable membrane (11) that retains liquid but allows vapour to escape.

4. A method of providing in usable form by means of a single apparatus a multiplicity of different substances that are advantageously disseminated or applied after undergoing a heat-induced change of state or fluidification, comprising the provision of each substance in a container (8) that comprises a secondary induction coil (13), the container being adapted to be used in association with an apparatus provided with a primary induction coil (2), the primary and secondary induction coils being in a heat-providing relationship when the container is in association with the apparatus, and providing electricity to the primary induction coil to generate sufficient heat in the secondary induction coil to induce in the liquid a desired change of state or fluidification, sufficient heat for the desired change in each individual substance being generated by moving one induction coil relative to the other.

5. A method according to claim 4, in which a state- or fluidification-inducing heating relationship of the primary and secondary coils for an individual substance is achieved by providing a fixed primary coil and, within each container, a secondary coil whose nature and/or placement is such that the degree of state- or fluidification-inducing heating appropriate to the substance is obtained.

6. An apparatus adapted to heat substances that advantageously undergo a heat-induced change of state or fluidification for useful application, the apparatus comprising a multiplicity of removable and replaceable containers (8), each holding a different substance with a different heating requirement, each container additionally comprising a secondary induction coil (13), heating being effected by electromagnetic induction by means of a primary induction coil (2) located in another part of the apparatus.

7. An apparatus according to claim 6, in which the primary induction coil apparatus is movable to permit with the secondary coil the attainment of the heating required for the particular substance.

8. An apparatus according to claim 6, in which the secondary induction coil of each container is of a suitable size and/or position such that, in combination with the primary coil, , the state- or fluidification-inducing heating appropriate to the particular substance is provided.

## Patentansprüche

1. Verfahren zum Abgeben eines Stoffs (10), wohin er benötigt wird, der vorteilhaft nach dem Durchlaufen einer wärmeinduzierten Zustandsänderung oder Fluidisierung abgegeben oder angewendet wird, wobei die Erwärmung durch Induktionswärmen durch Zuführen von Elektrizität zu einer primären Induktionsspule (2) durchgeführt wird, um Zustands- oder Fluidisierungsinduzierende Wärme in einer sekundären Induktionsspule (13) zu erzeugen, **dadurch gekennzeichnet, dass** der Stoff in einem Behälter (8) bereitgestellt wird, der die sekundäre Induktionsspule umfasst, wobei der Behälter entfernbar oder ersetzbar auf einer Basis (1) sitzt, die die primäre Induktionsspule umfasst, wobei die Spulen in einer wärmeliefernden Beziehung stehen, wenn der Behälter auf der Basis sitzt.

2. Verfahren gemäß Anspruch 1, wobei der Behälter mit einem eingezogenen Teil (12) versehen ist und die Basis ein nach oben ragendes Element (3) umfasst, das in einer behälterhaltenden Beziehung in den eingezogenen Teil passt.

3. Verfahren gemäß Anspruch 1, wobei der Stoff verdampfbar ist und der Behälter eine halbdurchlässige Membran (11) umfasst, die Flüssigkeit zurückhält aber Entweichen von Dampf erlaubt.

4. Verfahren zum Bereitstellen einer Vielzahl verschiedener Stoffe, die vorteilhaft nach dem Durchlaufen einer wärmeinduzierten Zustandsänderung oder Fluidisierung abgegeben oder angewendet werden, in verwendbarer Form mithilfe einer einzigen Vorrichtung, umfassend das Bereitstellen jedes Stoffs in einem Behälter (8), der eine sekundäre Induktionsspule (13) umfasst, wobei der Behälter dafür ausgelegt ist, in Verbindung mit einer Vorrichtung verwendet zu werden, die mit einer primären Induktionsspule (2) ausgestattet ist, wobei die primäre und die sekundäre Induktionsspule in einer wärmeliefernden Beziehung stehen, wenn der Behälter mit der Vorrichtung verbunden ist, und Zuführen von Elektrizität zu der primären Induktionsspule, um in der sekundären Induktionsspule ausreichend Wärme zu erzeugen, um in der Flüssigkeit eine gewünschte Zustandsänderung oder Fluidisierung zu induzieren, wobei in jedem einzelnen Stoff für die gewünschte Änderung ausreichende Wärme induziert wird, die erzeugt wird, indem eine Induktionsspule relativ zu der anderen bewegt wird.

5. Verfahren gemäß Anspruch 4, wobei eine Zustands- oder Fluidisierungs-induzierende wärmende Beziehung der primären und der sekundären Spule für einen einzelnen Stoff durch Bereitstellen einer feststehenden Primärspule und in jedem Behälter einer Sekundärspule erzielt wird, deren Beschaffenheit und/oder Anordnung so ist, dass das für den Stoff geeignete Ausmaß an Zustands- oder Fluidisierungs-induzierender Erwärmung erhalten wird.

6. Vorrichtung, dafür ausgelegt, Stoffe zu erwärmen, die vorteilhaft eine wärmeinduzierte Zustandsänderung oder Fluidisierung erfahren, für die nützliche Anwendung, wobei die Vorrichtung eine Vielzahl von beweglichen und ersetzbaren Behältern (8) umfasst, die jeweils einen anderen Stoff mit einem anderen Wärmebedarf enthalten, wobei jeder Behälter zusätzlich eine sekundäre Induktionsspule (13) umfasst, wobei das Erwärmen durch elektromagnetische Induktion mithilfe einer primären Induktionsspule (2) durchgeführt wird, die in einem anderen Teil der Vorrichtung angeordnet ist.

7. Vorrichtung gemäß Anspruch 6, wobei die Vorrichtung mit der primären Induktionsspule beweglich ist, um zu erlauben, dass das für den jeweiligen Stoff erforderliche Erwärmen mit der sekundären Induktionsspule durchgeführt wird.

8. Vorrichtung gemäß Anspruch 6, wobei die sekundäre Induktionsspule jedes Behälters eine geeignete Größe und/oder Position aufweist, so dass in Kombination mit der primären Spule das für den jeweiligen Stoff geeignete Zustands- oder Fluidisierungs-induzierende Erwärmen erzielt wird.

## Revendications

1. Procédé pour disséminer, là où cela est requis, une substance (10) qui est avantageusement disséminée ou appliquée après avoir subi une fluidisation ou un changement d'état induit par la chaleur, la chaleur étant fournie par un chauffage par induction, par fourniture d'électricité à une bobine d'induction primaire (2) pour générer de la chaleur induisant une fluidification ou un état dans une bobine d'induction secondaire (13), **caractérisé en ce que** la substance est disposée dans un récipient (8) qui comprend la bobine d'induction secondaire, le récipient étant placé de façon amovible ou remplaçable sur une base (1) qui comprend la bobine d'induction primaire, les bobines étant en relation de fourniture de chaleur quand le récipient est placé sur la base.

2. Procédé selon la revendication 1, dans lequel le récipient est doté d'une partie rentrante (12) et la base comprend un élément s'étendant vers le haut (3) qui s'ajuste dans la partie rentrante en relation de maintien du récipient.

3. Procédé selon la revendication 1, dans lequel la substance est évaporable et le récipient comprend une membrane semi-perméable (11) qui retient les liquides mais qui permet à la vapeur de s'échapper.

4. Procédé pour fournir sous forme utilisable, au moyen d'un seul dispositif, une multiplicité de substances différentes qui sont avantageusement disséminées ou appliquées après avoir subi une fluidification ou un changement d'état induit par la chaleur, comprenant la disposition de chaque substance dans un récipient (8) qui comprend une bobine d'induction secondaire (13), le récipient étant adapté pour être utilisé en association avec un dispositif doté d'une bobine d'induction primaire (2), les bobines d'induction primaire et secondaire étant en relation de fourniture de chaleur quand le récipient est en association avec le dispositif, et la fourniture d'électricité à la bobine d'induction primaire pour générer suffisamment de chaleur dans la bobine d'induction secondaire pour induire dans le liquide une fluidification ou un changement d'état souhaité, la chaleur suffisante pour le changement souhaité dans chaque substance individuelle étant générée par déplacement d'une bobine d'induction par rapport à l'autre.

5. Procédé selon la revendication 4, dans lequel une relation de chauffage induisant une fluidification ou un état des bobines primaire et secondaire pour une substance individuelle est obtenue par disposition d'une bobine primaire fixe et, dans chaque récipient, d'une bobine secondaire dont la nature et/ou le placement sont tels que le degré de chaleur induisant une fluidification ou un état approprié pour la substance soit obtenu.

6. Dispositif adapté pour chauffer des substances qui subissent avantageusement une fluidification ou un changement d'état induit par la chaleur pour une application utile, le dispositif comprenant une multiplicité de récipients amovibles et remplaçables (8), contenant chacun une substance différente ayant une exigence de chauffage différente, chaque récipient comprenant de plus une bobine d'induction secondaire (13), le chauffage étant effectué par induction électromagnétique au moyen d'une bobine d'induction primaire (2) située dans une autre partie du dispositif.

7. Dispositif selon la revendication 6, dans lequel le dispositif à bobine d'induction primaire est mobile pour permettre à la bobine secondaire d'atteindre le chauffage requis pour la substance particulière.

8. Dispositif selon la revendication 6, dans lequel la bobine d'induction secondaire de chaque récipient a une taille et/ou une position convenables de façon qu'en combinaison avec la bobine primaire, le chauffage induisant une fluidification ou un état approprié pour la substance particulière soit fourni.
